(19) Europäisches Patentamt / European Patent Office / Office européen des brevets

(11) **EP 3 283 855 B1**

(12) **EUROPEAN PATENT SPECIFICATION**

(45) Date of publication and mention
of the grant of the patent:
**23.06.2021 Bulletin 2021/25**

(21) Application number: **16779378.5**

(22) Date of filing: **18.04.2016**

(51) Int Cl.:
*G01F 1/76* (2006.01)     *A61M 15/00* (2006.01)
*A61M 16/20* (2006.01)     *G01P 5/26* (2006.01)
*G01F 25/00* (2006.01)     *G01F 1/86* (2006.01)
*G01F 15/075* (2006.01)     *G01F 1/708* (2006.01)

(86) International application number:
**PCT/CA2016/050446**

(87) International publication number:
**WO 2016/165029 (20.10.2016 Gazette 2016/42)**

(54) **INHALATION APPARATUS COMPRISING AN INHALATION CHAMBER AND AN OPTICAL FLOW MEASURING APPARATUS**

INHALATIONSVORRICHTUNG AUFWEISEND EINEN INHALATIONSRAUM UND EINE OPTISCHE DURCHFLUSSMESSVORRICHTUNG

APPAREIL D'INHALATION COMPRENANT UNE CHAMBRE D'INHALATION ET UN APPAREIL DE MESURE OPTIQUE DU DÉBIT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **17.04.2015 US 201562149019 P**
**26.05.2015 US 201562166225 P**
**28.09.2015 US 201562233524 P**

(43) Date of publication of application:
**21.02.2018 Bulletin 2018/08**

(73) Proprietor: **Protecsom Amerique du Nord Inc.**
**Drummondville, Québec J2C 7V3 (CA)**

(72) Inventors:
• **VERJUS, Romuald**
  **50100 Cherbourg (FR)**
• **POREE, Thierry**
  **50330 Saint-Pierre-Eglise (FR)**
• **GUHEL, Yannick**
  **50100 Cherbourg (FR)**
• **POUDEROUX, Bertrand**
  **50100 Cherbourg (FR)**
• **BOUDART, Bertrand**
  **50120 Equeurdreville (FR)**
• **ANGILELLA, Jean-Regis**
  **50000 Saint-Lo (FR)**

(74) Representative: **Cabinet Le Guen Maillet**
**3, impasse de la Vigie**
**CS 71840**
**35418 Saint-Malo Cedex (FR)**

(56) References cited:
**WO-A1-2005/088415     WO-A1-2007/031728**
**WO-A1-2007/066113     WO-A2-02/36190**
**US-A- 5 691 483     US-A1- 2004 011 975**
**US-A1- 2009 103 073     US-A1- 2013 008 436**

## Description

### CROSS-REFERENCE TO RELATED APPLICATIONS

This application claims priority of US provisional patent application No. 62/149,019, filed April 17, 2015, and US provisional patent application No. 62/166,225, filed May 26, 2015, and US provisional patent application No. 62/233,524, filed September 28, 2015.

### BACKGROUND

#### (a) Field

The subject matter disclosed generally relates to a flow measuring apparatus for the detection of particles, the determination of their mass and their velocity. More particularly, the subject matter relates to a flow measuring apparatus and inhalation apparatus for drug delivery by inhalation comprising the same.

#### (b) Related Prior Art

The ability to ensure that a drug substance reaches the lungs after inhalation therapy is a topic whose interest has grown in the last ten years. Indeed, the administration of drugs by inhalation is common in respiratory diseases such as asthma, cystic fibrosis, chronic obstructive pulmonary disease.

The effectiveness of the inhalation treatment is often proportional with the dose of drug substance reaching the area to be treated. Thus, the proper use of pressurized metered dose inhalers (MDI) and their derivatives is imperative.

Pressurized metered dose inhalers use a propellant which generates an aerosol of the active drug substance. Many patients using this type of device are unable to adequately coordinate their inspiration with actuation of the inhaler, which results in poor dosing of the active drug substance. This leads to the deposition of all the particles of the drug substance in the mouth and throat of the user, which is clearly undesirable.

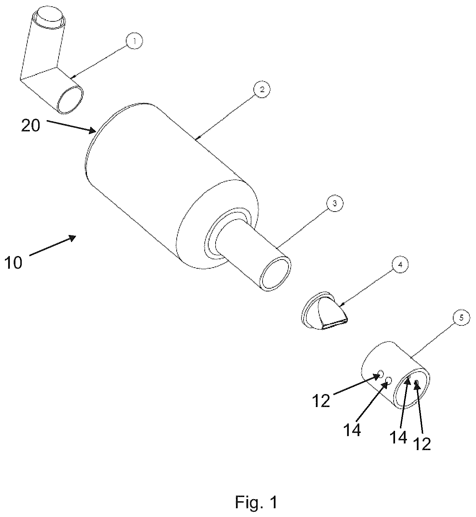

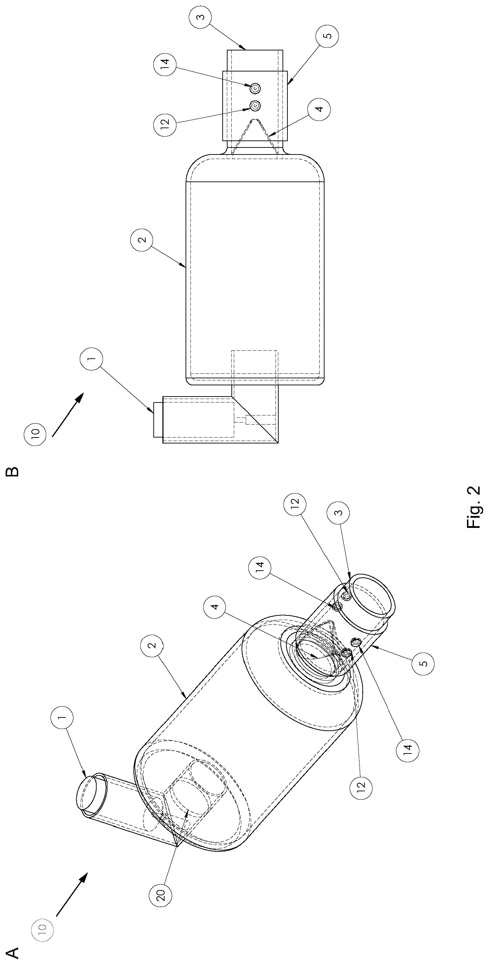

This problem can be solved by using an inhalation chamber (2), such as pictured in Figs. 1 and 2. This device consists of a closed inhalation chamber (2) connected to the MDI (1). A second end (3), commonly a mouthpiece is attached to the other end of the inhalation chamber (2) and contains a flexible inhalation valve (4) which opens when the patient inhales and closes when he/she expires. The inhalation chamber (2) plays the role of drug reservoir which empties when the patient inhales. Thus, the velocities of the drug substance particles are reduced at the mouth and therefore the losses of drug substance by impaction are reduced in the upper respiratory tract. In addition, this device allows a better deposition of the drug substance in the targeted zone of action of the drug (the alveolar zone) compared to a single metered dose inhaler.

However, it is difficult for the patient to ascertain that the inspiratory flow is efficient enough to inhale the drug. This information is important because too low or too high a flow can negatively affect treatment, because it may results in providing too little or even no drug, or too much of the drug. It is even more crucial when the drug must be ingested by young children because their inspiratory cycle is erratic and is characterized by relatively low flow rates. Indeed, nothing today allows the verification of the intake of inhaled medication.
The patent US 2013/0008436 discloses a respiraoty drug delivery aparatus including sensors for detecting to sens parameter relating to the use of the aparatus. For exemple, the presence of particles is detected by light sensors. However, the location of the measurement is not suitable.

Therefore, it is an object of the present invention to provide a flow measuring apparatus for measuring a flow of particles through a section of a fluid dispensing apparatus, or order to determine the presence of administered drug in the flow of particles, the mass (i.e. amount) of drug present in the flow of particles, and the velocity (speed) of said particles in the flow of particles. It is also an object of the present invention to provide an inhalation apparatus for drug delivery by inhalation, having a flow measuring apparatus for measuring a flow of drug particles through a section of said inhalation apparatus.

According to the invention, there is provided :
An inhalation apparatus comprising an inhalation chamber and a flow measuring apparatus for measuring a flow of particles from the inhalation chamber, the inhalation chamber having a first end to be connected to the source of drug, such as a metered drug inhaler, to be administered by inhalation do a user, and a second end to be connected to the user, through a section of an inhalation apparatus, said section being located between the second end of the inhalation chamber and the user, the flow measuring apparatus being located in the section of the inhalation apparatus and comprising:

- a sensor comprising at least one photo-detection device comprising a light source and a detector, wherein the light source may be configured to emit electromagnetic radiation onto the detector along a light path, wherein the detector may be configured to measure disturbance of the electromagnetic radiation by said flow of particles from the inhalation chamber to provide measurements of the presence of particles within said section of the inhalation apparatus and measurements of a mass of the particles flowing through the inhalation apparatus.

The flow measuring apparatus of the present invention may comprise:

- a sensor comprising at least two photo-detection device, each photo-detection device comprising a light source and a detector, wherein the light source may be configured to emit electromagnetic radiation onto the detector along a light path, wherein the detector may be configured to measure disturbance of the electromagnetic radiation by the flow of particles; and wherein the light paths are parallel, and at least one of the light paths may be inverted with respect to the other light paths.

The flow measuring apparatus of the present invention may comprise at least a first and a second sensor, the first sensor configured to measure the flow of particles along a first axis, and the second sensor configured to measure the flow of particles along a second axis perpendicular to the first axis.

The flow measuring apparatus may further comprise a processor operatively connected to the sensor for processing a measured disturbance of the electromagnetic radiation.

The processor may be operatively connected to the sensor for controlling one or more of a wavelength, a frequency, or an intensity of the electromagnetic radiation emitted by the light source.

The light source emits at wavelength from about 400 nm to about 1 mm.

The light source emits at wavelength from about 450 nm to about 600 nm.

One of the photo-detection device has a light source emitting at wavelength from <600 nm and another photo-detection device has a light source emitting at wavelength >600 nm.

The flow measuring apparatus may further comprise a visual means, operatively connected to the processor, for providing information about the flow of particles.

The visual means may be an indicator light, a display means, or combinations thereof.

The flow measuring apparatus may further comprise a transmission means, operatively connected to the processor, for transmitting information about the flow of particles to an external device.

The processor may be operative to provide a rate of drug delivery.

The visual means may be mounted on the flow measuring apparatus.

The visual means and the processor are configured to provide additional information regarding use of the flow measuring apparatus.

The visual means may be a detachable display.

The display means may be a plate comprising a visual information, an illuminated plate comprising a visual information, an LCD display, or a combination thereof.

The detachable display may be configured to be attached to a separate base.

The separate base may be a powered base, to power the detachable display.

The detachable base further comprise any one of a processor, memory, storage, input interface, and output interface.

The base may be integral to the detachable display.

According to another embodiment, there may be provided an inhalation apparatus for drug delivery by inhalation comprising :

- an inhalation chamber having
  a first end to be connected to a source of drug to be administered by inhalation to a user,
  a second end to be connected to the user;

- a valve, upstream of the inhalation chamber and downstream of the second end, allowing passage of a flow of drug particles from the inhalation chamber to the second end, and

- a flow measuring apparatus of the present invention, upstream of the valve, for measuring flow of the flow of drug particles.

The inhalation apparatus may further comprise an expiration conduit downstream of the second end, for exit of air expired by the user.

The expiration conduit may further comprise an expiration sensor comprising a photo-detection device comprising a light source and a detector, wherein the light source may be configured to emit electromagnetic radiation of wavelength > 600 nm onto the detector along a light path, wherein the detector may be configured to measure disturbance of the electromagnetic radiation by mist formation from the expired air from the user.

The inhalation apparatus may further comprise an upstream sensor, upstream of the valve, and comprising a photo-detection device comprising an upstream light source and an upstream detector, wherein the upstream light source may be configured to emit electromagnetic radiation of wavelength >600 nm onto the detector along a light path, wherein the upstream detector may be configured to measure disturbance of the electromagnetic radiation by the flow of particles to provide measurements of the presence and mass of particles within an upstream section of the inhalation apparatus.

According to another embodiment, not being part of the invention, there may be provided an method for the administration of a drug by inhalation to a subject in need thereof, comprising administering the drug with an inhalation apparatus according to the present invention.

The method may further comprise the step of measuring a disturbance of an electromagnetic radiation emitted from the sensor caused formation of a mist or a fog caused by exhalation of the user in the inhalation apparatus.

The method may further comprise the step of synchronizing administration of the drug with a decrease of the disturbance of an electromagnetic radiation emitted from the sensor caused by inhalation of the subject in the inhalation apparatus.

The method may further comprise the step of measuring a flow of particles.

The measuring includes at least one of measuring the presence of the flow of particles, measuring the mass of the flow of particles, measuring the velocity of the flow of particles, or combinations thereof.

The method may further comprise the step of generating a notification in view of the measuring a flow of particles.

The notification may be audible, visible, or both.

The velocity of the flow of particles may be at or above a threshold rate, the notification may be representative of a successful administration.

The velocity of the flow of particles may be below a threshold rate, the notification may be representative of an unsuccessful administration.

According to another embodiment, not being part of the present invention, there may be provided a use of an inhalation apparatus according to the present invention for the administration of a drug by inhalation to a subject in need thereof.

According to the present invention, there is provided an inhalation apparatus for the administration of a drug by inhalation to a subject in need thereof.

The following terms are defined below.

The term "light source" is intended to mean any suitable source of electromagnetic radiation which may be detected by an attendant detector. For example, suitable sources of lights may be light emitting diodes (LED) or a laser diode, emitting electromagnetic radiation which will be detected by the opposed detector (14). The wavelength of the electromagnetic radiation used may be any suitable wavelength, for example from about 100 nm to about 1 mm, or from about 400 nm to about 1 mm, or from about 500 nm to about 1 mm, or from about 600 nm to about 1 mm, or from about 700 nm to about 1 mm, or from about 800 nm to about 1 mm, or from about 900 nm to about 1 mm, or from about 1 $\mu$m to about 1 mm, or from about 10 $\mu$m to about 1 mm, or from about 100 $\mu$m to about 1 mm, or from about 200 $\mu$m to about 1 mm, or from about 300 $\mu$m to about 1 mm, or from about 400 $\mu$m to about 1 mm, or from about 500 $\mu$m to about 1 mm, or from about 600 $\mu$m to about 1 mm, or from about 700 $\mu$m to about 1 mm, or from about 800 $\mu$m to about 1 mm, or from about 900 $\mu$m to about 1 mm, from about 100 nm to about 900 $\mu$m, or from about 400 nm to about 900 $\mu$m, or from about 500 nm to about 900 $\mu$m, or from about 600 nm to about 900 $\mu$m, or from about 700 nm to about 900 $\mu$m, or from about 800 nm to about 900 $\mu$m, or from about 900 nm to about 900 $\mu$m, or from about 1 $\mu$m to about 900 $\mu$m, or from about 10 $\mu$m to about 900 $\mu$m, or from about 100 $\mu$m to about 900 $\mu$m, or from about 200 $\mu$m to about 900 $\mu$m, or from about 300 $\mu$m to about 900 $\mu$m, or from about 400 $\mu$m to about 900 $\mu$m, or from about 500 $\mu$m to about 900 $\mu$m, or from about 600 $\mu$m to about 900 $\mu$m, or from about 700 $\mu$m to about 900 $\mu$m, or from about 800 $\mu$m to about 900 $\mu$m, from about 100 nm to about 800 $\mu$m, or from about 400 nm to about 800 $\mu$m, or from about 500 nm to about 800 $\mu$m, or from about 600 nm to about 800 $\mu$m, or from about 700 nm to about 800 $\mu$m, or from about 800 nm to about 800 $\mu$m, or from about 900 nm to about 800 $\mu$m, or from about 1 $\mu$m to about 800 $\mu$m, or from about 10 $\mu$m to about 800 $\mu$m, or from about 100 $\mu$m to about 800 $\mu$m, or from about 200 $\mu$m to about 800 $\mu$m, or from about 300 $\mu$m to about 800 $\mu$m, or from about 400 $\mu$m to about 800 $\mu$m, or from about 500 $\mu$m to about 800 $\mu$m, or from about 600 $\mu$m to about 800 $\mu$m, or from about 700 $\mu$m to about 800 $\mu$m, from about 100 nm to about 700 $\mu$m, or from about 400 nm to about 700 $\mu$m, or from about 500 nm to about 700 $\mu$m, or from about 600 nm to about 700 $\mu$m, or from about 700 nm to about 700 $\mu$m, or from about 800 nm to about 700 $\mu$m, or from about 900 nm to about 700 $\mu$m, or from about 1 $\mu$m to about 700 $\mu$m, or from about 10 $\mu$m to about 700 $\mu$m, or from about 100 $\mu$m to about 700 $\mu$m, or from about 200 $\mu$m to about 700 $\mu$m, or from about 300 $\mu$m to about 700 $\mu$m, or from about 400 $\mu$m to about 700 $\mu$m, or from about 500 $\mu$m to about 700 $\mu$m, or from about 600 $\mu$m to about 700 $\mu$m, from about 100 nm to about 600 $\mu$m, or from about 400 nm to about 600 $\mu$m, or from about 500 nm to about 600 $\mu$m, or from about 600 nm to about 600 $\mu$m, or from about 700 nm to about 600 $\mu$m, or from about 800 nm to about 600 $\mu$m, or from about 900 nm to about 600 $\mu$m, or from about 1 $\mu$m to about 600 $\mu$m, or from about 10 $\mu$m to about 600 $\mu$m, or from about 100 $\mu$m to about 600 $\mu$m, or from about 200 $\mu$m to about 600 $\mu$m, or from about 300 $\mu$m to about 600 $\mu$m, or from about 400 $\mu$m to about 600 $\mu$m, or from about 500 $\mu$m to about 600 $\mu$m, from about 100 nm to about 500 $\mu$m, or from about 400 nm to about 500 $\mu$m, or from about 500 nm to about 500 $\mu$m, or from about 600 nm to about 500 $\mu$m, or from about 700 nm to about 500 $\mu$m, or from about 800 nm to about 500 $\mu$m, or from about 900 nm to about 500 $\mu$m, or from about 1 $\mu$m to about 500 $\mu$m, or from about 10 $\mu$m to about 500 $\mu$m, or from about 100 $\mu$m to about 500 $\mu$m, or from about 200 $\mu$m to about 500 $\mu$m, or from about 300 $\mu$m to about 500 $\mu$m, or from about 400 $\mu$m to about 500 $\mu$m, from about 100 nm to about 400 $\mu$m, or from about 400 nm to about 400 $\mu$m, or from about 500 nm to about 400 $\mu$m, or from about 600 nm to about 400 $\mu$m, or from about 700 nm to about 400 $\mu$m, or from about 800 nm to about 400 $\mu$m, or from about 900 nm to about 400 $\mu$m, or from about 1 $\mu$m to about 400 $\mu$m, or from about 10 $\mu$m to about 400 $\mu$m, or from about 100 $\mu$m to about 400 $\mu$m, or from about 200 $\mu$m to about 400 $\mu$m, or from about 300 $\mu$m to about 400 $\mu$m, from about 100 nm to about 300 $\mu$m, or from about 400 nm to about 300 $\mu$m, or from about 500 nm to about 300 $\mu$m, or from about 600 nm to about 300 $\mu$m, or from about 700 nm to about 300 $\mu$m, or from about 800 nm to about 300 $\mu$m, or from about 900 nm to about 300 $\mu$m, or from about 1 $\mu$m to about 300 $\mu$m, or from about 10 $\mu$m to about 300 $\mu$m, or from about 100 $\mu$m to about 300 $\mu$m, or from about 200 $\mu$m to about 300 $\mu$m, from about 100 nm to about 200 $\mu$m, or from about 400 nm to about 200 $\mu$m, or from about 500 nm to about

200 μm, or from about 600 nm to about 200 μm, or from about 700 nm to about 200 μm, or from about 800 nm to about 200 μm, or from about 900 nm to about 200 μm, or from about 1 μm to about 200 μm, or from about 10 μm to about 200 μm, or from about 100 μm to about 200 μm, from about 100 nm to about 100 μm, or from about 400 nm to about 100 μm, or from about 500 nm to about 100 μm, or from about 600 nm to about 100 μm, or from about 700 nm to about 100 μm, or from about 800 nm to about 100 μm, or from about 900 nm to about 100 μm, or from about 1 μm to about 100 μm, or from about 10 μm to about 100 μm, from about 100 nm to about 10 μm, or from about 400 nm to about 10 μm, or from about 500 nm to about 10 μm, or from about 600 nm to about 10 μm, or from about 700 nm to about 10 μm, or from about 800 nm to about 10 μm, or from about 900 nm to about 10 μm, or from about 1 μm to about 10 μm, from about 100 nm to about 1 μm, or from about 400 nm to about 1 μm, or from about 500 nm to about 1 μm, or from about 600 nm to about 1 μm, or from about 700 nm to about 1 μm, or from about 800 nm to about 1 μm, or from about 900 nm to about 1 μm, from about 100 nm to about 900 nm, or from about 400 nm to about 900 nm , or from about 500 nm to about 900 nm, or from about 600 nm to about 900 nm, or from about 700 nm to about 900 nm, or from about 800 nm to about 900 nm, from about 100 nm to about 800 nm, or from about 400 nm to about 800 nm , or from about 500 nm to about 800 nm, or from about 600 nm to about 800 nm, or from about 700 nm to about 800 nm, from about 100 nm to about 700 nm, or from about 400 nm to about 700 nm , or from about 500 nm to about 700 nm, or from about 600 nm to about 700 nm, from about 100 nm to about 600 nm, or from about 400 nm to about 600 nm , or from about 500 nm to about 600 nm, from about 100 nm to about 500 nm, or from about 400 nm to about 500 nm , from about 100 nm to about 400 nm, and preferably from between about 400 nm to about 1 mm, and also from about 200 nm to about 1 mm, and also 450 nm to about 600 nm, or about 450 nm to about 700 nm, or about 450 nm to about 800 nm, or about 450 nm to about 900 nm, or about 450 nm to about 1 mm, according to the fluid flowing in the apparatus (e.g. a suspension of a drug).

The term "detector" is intended to mean photosensors or photodetectors which are sensors of light or other electromagnetic energy, which measure the electromagnetic radiation emitted by the light source. Suitable detectors include but are not limited to photo-resistors, photodiodes, or phototransistors.

The term "fluid" is intended to mean a substance that continually deforms (flows) under an applied shear stress. Fluids are a subset of the phases of matter and include liquids, gases, plasmas and, to some extent, plastic solids. Fluids can be defined as substances that have zero shear modulus or in simpler terms a fluid is a substance which cannot resist any shear force applied to it. According to embodiments, preferred fluids are liquids and gases, more preferably gases, and especially gasses having particles of medication or drugs suspended therein. According to an embodiment, the gas may be gaseous discharge containing dry powder of a medicamentous substance found in a metered dose inhaler for example.

The term "photo-detection device" is intended to mean a device comprising a light source and a detector to measure the electromagnetic radiation emitted by the light source.

The term "inhalation apparatus" is intended to mean a medical device used for delivering medication into the body via the lungs. This includes metered-dose inhalers, dry powder inhalers, nebulizers and nasal inhalers, for example.

The term "mass", or "measured mass" or "total mass" is intended to mean the quantity, amount, of particles of the drug particles that flowed through the section of the fluid dispensing apparatus. The measurement of the mass of drug particle allows the determination of the actual dose inhaled by the subject, and allows determining if the subject is getting an adequate or inadequate treatment.

Features and advantages of the subject matter hereof will become more apparent in light of the following detailed description of selected embodiments, as illustrated in the accompanying figures. As will be realized, the subject matter disclosed and claimed is capable of modifications in various respects, all without departing from the scope of the claims. Accordingly, the drawings and the description are to be regarded as illustrative in nature, and not as restrictive and the full scope of the subject matter is set forth in the claims.

## BRIEF DESCRIPTION OF THE DRAWINGS

Further features and advantages of the present disclosure will become apparent from the following detailed description, taken in combination with the appended drawings, in which:

Fig. 1 illustrates an inhalation apparatus (exploded view) according to an embodiment of the present invention;

Fig. 2 illustrates an inhalation apparatus (global view) according to an embodiment of the present invention;

Fig. 3A illustrates a photo-detection device as used in a flow measuring apparatus according to an embodiment of the present invention. The illustration shows the simplified principle of the optical sensor for determining the presence, amount and velocity of drug substance to the outlet of the inhaler (side view). F = flow of particles;

Fig. 3B illustrates the positioning of the optical path (arrow between the light source (emitter) and detector) to the aerosol jet (shown schematically by the blue dots). Configuration 1 (left): optical path in the plane of the particles jet. Configuration 2 (right): the optical path perpendicular to the plane of the particles jet. F = flow of particles;

Fig. 4A illustrates positioning light sources (emitters) and detectors on the mouthpiece of the second end (3) of the inhalation chamber (2) in the configuration 1. F = flow of particles;

Fig. 4B illustrates positioning light sources (emitters) and detectors on the mouthpiece of the second end (3) of the inhalation chamber (2) in the configuration 2. F = flow of particles;

Fig. 5A illustrates a top view of two photo-detection devices on a flow measuring apparatus (5) in configuration 1. They are used to determine the average speed of particles (here schematically represented by small circles). The arrows between the photo-detection device show the optical paths. These optical paths are of opposite directions in order to limit any interference flux received by the detectors. F = flow of particles;

Fig. 5B illustrates a cross-sectional view of two photo-detection devices on a flow measuring apparatus (5) in configuration 2. These determine the particle velocity profile in the center of the flow of particles. F = flow of particles;

Fig. 6 illustrates schematically the functioning of an inhalation apparatus according to an embodiment of the present invention.

Fig. 7A is a front view of an inhalation apparatus with a flow measuring apparatus of the present invention with a light source and display, according to an embodiment of the present invention.

Fig. 7B is a side view of an inhalation apparatus with a flow measuring apparatus of the present invention with a light source and display, according to an embodiment of the present invention.

Fig. 7C is a front view of an inhalation apparatus with a flow measuring apparatus of the present invention with a display according to an embodiment of the present invention.

Fig. 7D is a side view of an inhalation apparatus with a flow measuring apparatus of the present invention with a display according to an embodiment of the present invention.

Fig. 8 illustrates a combination of a display (42) and a base (50) according to an embodiment of the present invention.

Fig. 9A illustrates the intensity of a signal S(t) as a function of time, measured by a photo-detection device of the present invention in an apparatus of the present invention, during the passage of an aerosol. The detection threshold is indicated. When the signal reaches this value, the passage of the aerosol is detected.

Fig. 9B illustrates a signal measured with an infrared sensor in an inhalation chamber during a respiratory cycle. The signal increases during expiration due to fogging on the mouthpiece (element 3 on Fig. 1). During inhalation, the mist evaporates and the signal returns to its original position.

Fig. 10 illustrates an example of the mass of the corticosteroid fluticasone (50 $\mu$g per dose) measured as a function of the integral of the signal S(t) measured by the a photo-detection device of the present invention at the exit of an apparatus of the present invention. Legend: m = mass measure on filter, and I is the time integral of the signal S(t) measured with the photo-detection device. The line is the power function $m(S) = 0.34 \times I^{0.99}$ that fits the calibration curve. With this function, the sensor can determine the mass of fluticasone that is inhaled by the subject.

Fig. 11 illustrates the positioning of the photo-detection device with respect to the aerosol flow, according to an embodiment of the flow measuring apparatus of the present invention.

Fig. 12A illustrates the temporal signals intensity measured by each detector and recorded as S1(t) and S2(t).

Fig. 12B illustrates the error $E(T) = \int |S1(t) - S2(t + T)|^2 dt$, which is approximated by a discrete sum, and allows the determination of $T_0$.

It will be noted that throughout the appended drawings, like features are identified by like reference numerals.

## DETAILED DESCRIPTION

In an embodiment, not according to the present invention, there is disclosed a flow measuring apparatus (5) for measuring a flow of particles through a section of a fluid dispensing apparatus. According to an embodiment, not according to the present invention, there is disclosed a flow measuring apparatus for measuring a flow of particles through a section of a fluid dispensing apparatus which comprises a sensor which comprises at least one photo-detection device comprising a light source (12) and a detector (14), where the light source (12) is configured to emit electromagnetic radiation onto the detector (14) along a light path and the detector (14) is configured to measure disturbance of the electromagnetic radiation by the flow of particles to provide a measurement of the presence of particles within the section of the fluid dispensing apparatus and a measurement of a mass of the particles flowing in the section of a fluid dispensing apparatus. The detection of the presence of particles allows a more accurate counting of the number of doses administered, since some drug delivery devices (e.g. MDI) when not handled properly, only eject propellant gas without any drug particles. Furthermore, the detection of the mass or amount of drug particles administered allows the determination of an adequate or inadequate amount of drug particles has been delivered for the desired treatment.

Now referring to Figs. 1 and 2, the apparatus (5) comprises a sensor, which comprises at least two photo-detection devices. Each photo-detection device comprises a light source (12) and a detector (14). The light source (12) is configured to emit electromagnetic radiation onto the detector (14) along a light path (16), and the detector (14) is configured to measure disturbance of the electromagnetic radiation by the flow of particles flowing through the light path. The light paths (16) between each light source (12) and detector (14) pair are parallel to one another, and at least one of the light paths is inverted with respect to the other light paths. The sensor comprising at least two photo-detection devices allows the detection of the presence of particles, their total mass, and the velocity of the particles.

According to another embodiment, not according to the present invention, the flow measuring apparatus (5) for measuring a flow of particles through a section of a fluid dispensing apparatus may comprise at least a first sensor, configured to measure the flow of particles along a first axis, and a second sensor, configured to measure the flow of particles along a second axis perpendicular to the first axis. According to an embodiment, each sensor comprises at least one or at least two photo-detection devices. Each photo-detection device comprises a light source (12) and a detector (14). The light source (12) is configured to emit electromagnetic radiation onto the detector (14) along a light path (16), and the detector (14) is configured to measure disturbance of the electromagnetic radiation by the flow of particles. The light paths (16) between each light source (12) and detector (14) pair are parallel to one another, and at least one of the light paths is inverted with respect to the other light paths.

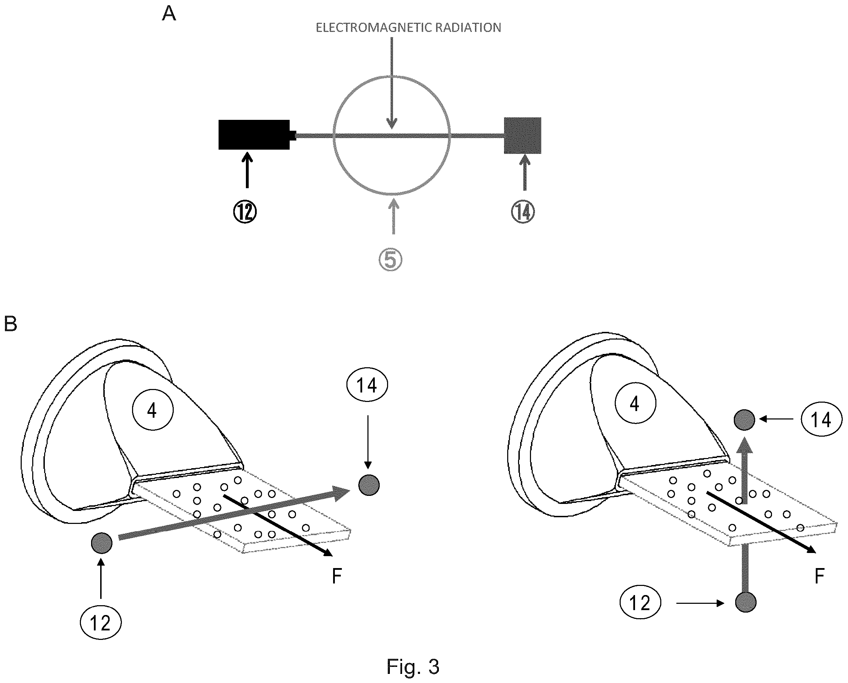

For example, the photo-detection device may comprise a light source (12) which may be for example a light emitting diode (LED) or a laser diode, emitting electromagnetic radiation which will be detected by an opposed detector (14) such as a photo-resistor, a photodiode, or a phototransistor, as shown schematically in Fig. 3A. The wavelength of the electromagnetic radiation used may be between any suitable wavelength, and for example the wavelength described above, particularly at about 200 nm to 1 mm, or about 400 nm to 1 mm (which includes the visible spectrum up to the far infrared), according to the suspension or the drug used. The infrared spectrum may be preferred, while according to other examples, visible light may be used. The wavelength of light may be from a range including more than one specific type of light (e.g. visible, infrared and ultraviolet). For example, according to some medicaments, these medicaments may be transported by droplets of propulsion gas that are capable to mostly or entirely absorb the infrared light. Limiting detection to this spectrum detects the droplets of propulsion gas, but does not detect the active pharmaceutical ingredient. Therefore, the use of visible light for example, instead of, or in combination with infrared light allows detection of the active pharmaceutical ingredient.

The photo-detector devices are placed on either side of the path of the flow of particles. This jet will disturb the electromagnetic radiation, and measurement and analysis of this disturbance will allow the determination of the amount of inhaled drug particles. Indeed, the passage of suspended particulates will cause a change in the intensity of the optical signal received by the detector, which will then emit a different amplitude electrical signal.

The apparatus of the present invention are non-invasive, that is to say that there is no contact between the apparatus and aerosols, so that it will not disturb the flow of particles.

The flow measuring apparatus (5) may be used to measure flow, including the presence, mass (i.e. amount) and velocity of particles having different shapes and configurations. The flow of particle may be a homogenous distribution of particles flowing through an enclosure (such as a tubular enclosure, or a relatively tubular enclosure). For example, the flow of particle may exit from a valve which leaves the flow homogenously distributed, or relatively so. The flow of particle may be a jet of particles flowing through an enclosure. The jet may be any type of jet, for example a narrow jet and uniform jet, a wide and uniform jet, or a jet having a shape imparted by an upstream element, such as a valve having an opening that may shape the flow of particle as in passes therethrough. For example, the flow of particle may be a relatively flat jet, for example at the exit of a duckbill valve (4) where the flow of aerosol particles forms a relatively flat jet. In cases where the flow of particles is relatively homogenously distributed within the enclosure, no particular configuration of the photo-detection device may be necessary, as the emitted electromagnetic radiation will interact with particles in approximately the same manner irrespective of the provenance of electromagnetic radiation. In cases where the flow of particles may be non-homogenously distributed within the enclosure, such as for the relatively flat jet described above, two configurations may be retained to make appropriate measurements, as illustrated in Fig. 3B: configuration 1 where the optical path (16) is in the plane of the jet, and configuration 2 where the optical path is perpendicular to the plane of the jet.

Both configurations yield different and complementary velocimetric information. The correlation between the two signals thus collected allows the determination of velocimetric information and throughput. This information may be obtained by cross-correlation of optical signals. The particle velocity at the outlet of the second end (3) and/or flexible inhalation valve (4) is not uniform. Indeed, it is fast in the center of the jet and slower at the edges. Flow measuring apparatus configured according to configuration 1 will determine an average velocity in the plane of the jet or flow (F), while those configured according to configuration 2 will give information on the velocity profile in the center of the jet.

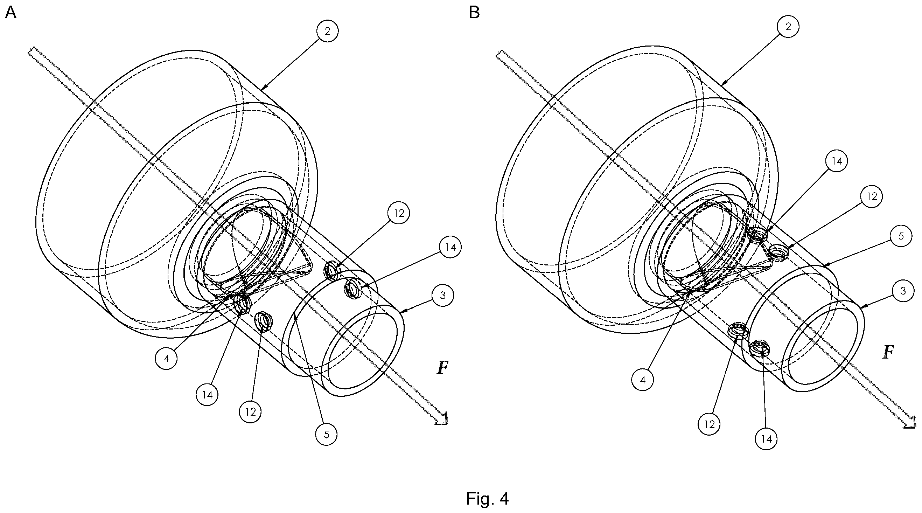

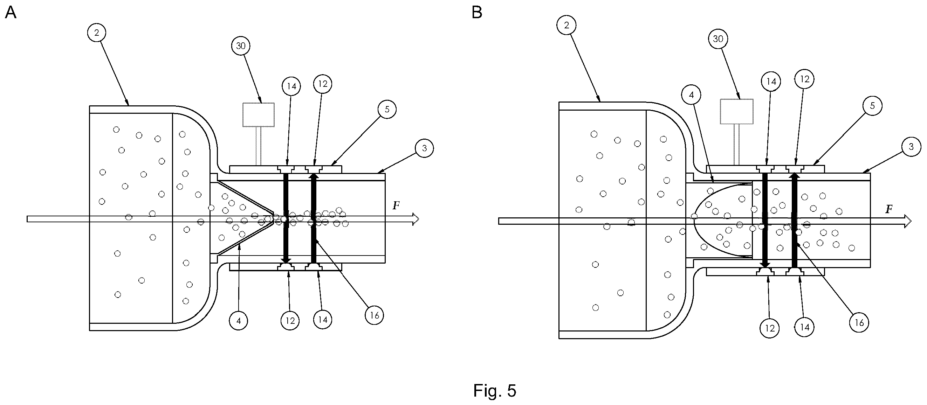

Figs. 4A and 4B show the position of two photo-detection device in the two configurations. To limit stray flux received by the detectors, the optical paths (16) of each photo-detection device will be opposite, as shown in Figs. 5A and 5B.

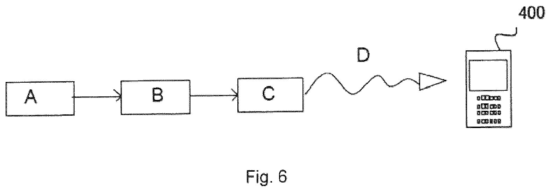

The photo-detection devices may be operatively connected to a processor (and/or controller) (30). The processor 30 may be both a processor and a controller, or a processor only, wit a separate device taking in charge any of the controllable features of the present apparatus. The term controller and processor, as used herein, are to be used interchangeably to identify a means of controlling aspects of the present invention in need of being controlled, and means of processing the harnessed information concerning the disturbances in the electromagnetic radiation measured by the detector (14). According to an example, the processor (30) is operatively connected to the sensor(s) of the flow measuring apparatus (5) for calculating the flows from the disturbances in the electromagnetic radiation measured by the detector. According to another example, the processor (30) may also be operatively coupled to the flow measuring apparatus (5) for controlling the wavelength of the electromagnetic radiation emitted by the light source (12), as well as frequency and intensity. The processor (30) may transmit a flow rate signal and/or activate visual means, such as a light emitting diode, or a display means indicating information concerning the measured flow, including the presence, mass (i.e. amount) and velocity of particles therein. The operation of these elements is described in Fig. 6.

According to the present invention, there is disclosed an inhalation apparatus (10) for drug delivery by inhalation. This inhalation apparatus comprises an inhalation chamber (2) having a first end (20) to be connected to a source of drug [such as MDI (1)] to be administered by inhalation to a user, and a second end (3) to be connected to the user. A valve (4) is disposed upstream of the inhalation chamber (2) and downstream of the second end (3), allowing passage of a flow of drug particles from the inhalation chamber (2) to the second end (3), and a flow measuring apparatus (5) of the present invention, as described above, upstream of the valve (4), for measuring flow, including the presence, mass (i.e.

amount) and velocity of drug particles. In embodiments of the present invention, the valve (4) may be any suitable valve that may be selected from and not limited to the wide selection of valve already known in the art, such as diaphragm valves, and duckbill valves.

In embodiments, the photo-detection devices of the flow measuring apparatus (5) may be operatively connected to a processor (30). The processor (30) may be operatively connected to a sensor of the flow measuring apparatus (5) for calculating the flows from the disturbances in the electromagnetic radiation measured by the detector. According to another embodiment, the processor (30) may also be operatively coupled to the sensor of the flow measuring apparatus (5) for controlling the wavelength of the electromagnetic radiation emitted by the light source (12), as well as frequency and intensity. The processor (30) may transmit a flow rate signal and/or activate audible and/or visual means, such as speakers, an indicator light (e.g.light emitting diode), or a display means indicating information concerning the measured flow and/or information about the proper use of the inhalation apparatus. The operation of these elements is described in Fig. 6.

Now referring to Fig. 6, the flow measuring apparatus (5) measures disturbance of the electromagnetic radiation by the flow of particles (step A). The processor (30) receives the optical signal received by the detector and calculates the flow rate (step B). The transmission means (32) transmits the signal flow delivered by the processor (30) to an external device 400, such as a portable machine equipped with a Bluetooth system or the likes (step D). This can be for example a cell phone, a tablet, etc. This allows the display on the external device (400) of either the flow value measured, or an indication to the user that a correct rate value and a proper functioning, or an incorrect rate value and an improper functioning of the inhalation apparatus (200).

According to another embodiment, it is also possible to transmit a signal for the rate of drug delivery from the processor (30) to a visual means attached to the inhalation apparatus (10) (step C) of Fig. 6 and/or to the flow measuring apparatus (5). The visual means may be for example a LED which illuminates when the value of the measured flow is the expected value or when on the contrary the value of the measured flow rate is less than a predetermined threshold value. This informs the user of the good or improper use or operation of the inhalation apparatus (10) and the smooth running of the treatment.

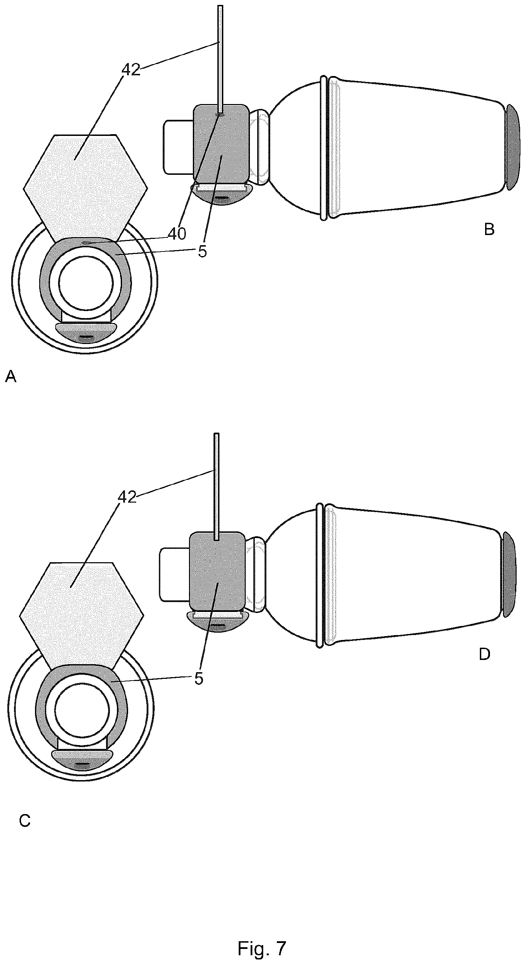

According to another embodiment and referring to Figs. 7A-B, the processor (30) may transmit a signal corresponding to the rate of drug delivery to a visual means which may comprise an indicator light (e.g. a LED) (40), operatively coupled to a display (42). According to embodiment, as shown in Figs. 7A-B, the indicator light (40) and display (42) may be mounted upon the flow measuring apparatus (5). For example the display (42) may be mounted through known means such as insertion in a slot, or any other suitable means. The display (42) may be a Plexiglas® plate, or an equivalent material. According to an embodiment, the plate may be etched or engraved with visual information upon which the light of the indicator light (40) may be reflected. In embodiments, the color and intensity of the light reflected upon the display (42) may be varied such that information concerning the flow measured by the flow measuring apparatus (5) may be communicated to the user. For example, for very young users, the indicator light (40) could display an assortment of lights on the display (42) designed at getting the user's attention when the flow measuring apparatus (5) is correctly or incorrectly used. According to another example, the indicator light (40) could display an assortment of lights on the display (42), thus illuminating the informative and/or amusing engraved visual information designed at getting the user's attention when the flow measuring apparatus (5) is correctly or incorrectly used. According to another example, the display (42) may also be used to display other information not related directly to the flow information measured, such as the status of an energy source electrically coupled to the apparatus (5), the number of doses administered and/or remaining in the source or drug coupled to the apparatus, etc., or any other information stored on attendant memory or storage means.

According to another embodiment and referring to Figs. 7C-D, the processor (30) of a flow measuring apparatus (5) may transmit a signal corresponding to the rate of drug delivery to a visual means such as a display (42). According to embodiment, as shown in Figs. 7C-D, the display (42) may be mounted upon the flow measuring apparatus (5), through known means such as insertion in a slot, or any other suitable means. The display (42) may be a liquid crystal display (LCD) or an equivalent device. According to an embodiment, the LCD may be used to display visual information obtained from the processor (30), corresponding to the status of the flow measuring apparatus, and/or other information stored on attendant memory or storage means. In embodiments, visual information upon the display (42) may be varied such that information concerning the flow measured by the flow measuring apparatus (5) may be communicated to the user. For example, for very young users, the visual information displayed upon the display (42) may be designed at getting the user's attention when the flow measuring apparatus (5) is correctly or incorrectly used. According to another example, the display (42) may also be used to display other information not related directly to the flow information measured, such as the status of an energy source electrically coupled to the apparatus (5), the number of doses administered and/or remaining in the source or drug coupled to the apparatus, etc., or any other information stored on attendant memory or storage means.

According to another embodiment, the display (42) may be a detachable display. According to one embodiment, the detachable display may be interchanged with another display. This detachable display may be advantageous feature to replace broken or damaged displays. Also, the apparatus of the present invention may be provided with alternative displays (42), or such additional alternative displays (42) could be purchased separately, to be used with the flow measuring apparatus (5) of the present invention. According to embodiments, the display (42) may be plates etched or engraved with differing visual information (e.g. different artistic images, different language information, and the likes) upon which a light source may be reflected. According to another embodiment, detachable display (42) may be a liquid crystal display (LCD) or an equivalent device, as described above. The LCD displays may be of various size and density, so as to suit a number of applications as may be necessary. For example, the LCD display may be of a relatively low quality to meet a specific price point, or it may be of a high quality and/or durability, so as to be used under professional conditions where the flow measuring apparatus (5) of the present invention could be used frequently by several distinct users.

According to embodiments, the detachable display (42) may be mounted (fastened) to the flow measuring apparatus (5) through conventional market methods of attaching two objects in a detachable manner. According to an embodiment, the detachable display (42) may be separated from separated from the flow measuring apparatus (5) when a certain pulling, twisting, pushing, or other force is applied to the the display (42) without any tools needed. This solves the problem that a user may apply too much torque to the flow measuring apparatus (5) when pulling out the detachable display (42) and as a result break the flow measuring apparatus (5). According to yet another embodiment, the detachable display (42) may be separated from separated from the flow measuring apparatus (5) when a certain pulling, twisting, pushing, or other force is applied to the the display (42), with an appropriate tool, and/or after fasteners holding the display (42) have been previously been detached with appropriate tools. This solves the problem that a user may inadvertently detach the display (42) when applying too much torque to the flow measuring apparatus (5).

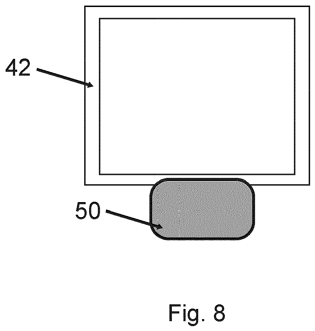

Now referring to Fig. 8, according to another embodiment, the detachable display (42) may be reattached to a separate base (50). According to an embodiment, the base (50) may be a simple base on which the unused display (42) may be placed to present the artwork found on the display (42). In another embodiment, the base (50) may be a powered base, comprising the necessary electrical means to power (e.g. externally or internally - wired or battery powered) and/or illuminate the display (42) attached thereto. For example, the base (50) may comprise the necessary electrical circuits to power one or more LED lights to illuminate the attached display (42), which could cycle through different colors and intensities. According to such an embodiment, the base (50) and display (42) may be used as a night light for a young child, which would associate the images displayed in the night light, along with some addible signals, with a positive use of the flow measuring apparatus (5) of the present invention.

According to another embodiment, the base (50) may comprise processor, memory, storage, output (e.g. speakers, lights, other displays) and interfacing (e.g. USB ports, Wi-Fi, and Bluetooth®) means, as well as the necessary electrical means to power an LCD based display (42) so as to store information onto the base (50) for display on the display (42). For example, the LCD based display could display pictures, videos, or information in succession, provide a visual and/or audio reminder or alarm at a predetermined time interval, etc., for use as a night light, information and entertainment device.

In another embodiment, the base (50) may be a powered base, comprising the necessary electrical means to power and/or illuminate the display (42) attached thereto. For example, the base (50) may comprise the necessary electrical circuits to power an attached display (42) and the attached display (42) may comprise an assortment of processor, memory, storage, output (e.g. speakers, other displays) and/or interfacing (e.g. USB, Wi-Fi, and Bluetooth®) means, to be powered by the electrical means in the base (50). Again, the information stored onto the storage of the display (42) could be displayed onto the display. For example, the LCD based display could display pictures, videos, or information in succession, provide a visual and/or audio reminder or alarm at a predetermined time interval, etc.

According to another embodiment, the base (50) may be integral (built in or built with) the display (42). For example, display (42) may comprise a flat surface or legs underneath it which may act as a base (50). In this embodiment, the display (42) may comprise an assortment of processor, memory, storage, output (e.g. speakers, other displays) and/or interfacing (e.g. USB, Wi-Fi, and Bluetooth®) means, and electrical means required to power it to illuminate the artwork on the display (42) and/or the LCD based display. Again, the information stored onto the storage of the display (42) could be displayed onto the display. For example, the LCD based display could display pictures, videos, or information in succession, provide a visual and/or audio reminder or alarm at a predetermined time interval, etc. According to another embodiment, the combined base (50) and display (42) may also comprise the necessary electronic means (e.g. a timer or clock function) to provide a visual and/or audio reminder or alarm at a predetermined time interval.

According to another embodiment, the interfacing means present in the display (42) and/or base (50) may also be used to communicate the collected information concerning the flow information measured, the status of an energy source electrically coupled to the apparatus (5), the number of doses administered and/or remaining in the source or drug coupled to the apparatus, etc., or any other information stored on attendant memory or storage means, to an external application. For example, the flow information measured, the number of doses administered and/or remaining in the source or drug coupled to the apparatus could be provided to a treating physician to monitor progress of the treatment. Other information stored on the apparatus (5) and/or on the base (50) could potentially be used for remote diagnostic of a failing apparatus (5), for example.

According to another embodiment, not being part of the present invention, there is also provided a method for the administration of a drug by inhalation to a subject in need thereof which comprises administering the drug with an inhalation apparatus according to the present invention. The method may further comprise the step of measuring a disturbance of the electromagetic radiation emitted from the sensor which may be caused when the subject using the apparatus exhales (expires) therein. Indeed, the exhalation of warm air from the lungs of the subject causes the formation of mist inside the inhalation apparatus, which may be detected by the sensor of the present invention.

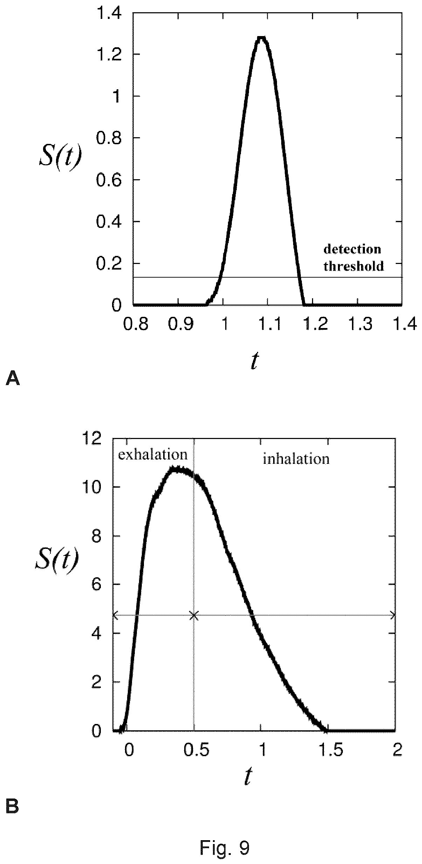

When the subect or patient exhales into the inhalation chamber, moisture is created on the walls of the mouthpiece (element 3 in Fig. 1). When the optical path has a wavelength >600 nm, a large portion of the emitted light will be absorbed by the mist and this will lead to a strong variation of the measured signal (see Fig. 9B). Inspiration will have the effect of removing mist from the mouthpiece. The signal will then return to its original position. In visible light of wavelength between 450 and 600 nm, the creation of vapor has very little effect on the signal measured. Indeed, the maximum amplitude of the signal related to fogging/misting is then well below the detection threshold. Using a photo-detection device having a light source having wavelength >600 nm with an inhalation chamber allows the detection of the inspirations and expirations of the patient. This has the advantage of allowing the user to know when to actuate the inhaler, the best time being during inspiration.

The fog detection with light >600 nm can be restrictive as the mist created in the mouthpiece can be confused with a passage of an aerosol. The use of at least two photo-detection device, at least one of which has light source having a wavelength >600 nm eliminates this problem for the detection of aerosols and allow detection of the patient's breathing. The possible configurations to ensure these two functions are as follows:

**a) photo-detection device having a light source of wavelength <600 nm and photo-detection device having a light source of wavelength of wavelength >600 nm version 1**

In this first embodiment, a first photo-detection device having a light source of wavelength <600 nm is used for measurements for aerosols, and a second photo-detection device having a light path of wavelength >600 nm is used for fog detection. For example, the configuration of the two optical paths may be that of Fig. 4.

The photo-detection devices are placed after the valve. The sensor as follows: During the passage of aerosols, the two signals reach their limit of detection. When the fog is produced into the mouthpiece, only the second photo-detection device having light source of wavelength >600 nm reaches the detection threshold.

**b) photo-detection device having a light source of wavelength <600 nm and photo-detection device having a light source of wavelength of wavelength >600 nm version 2**

According to a second embodiment, a second possibility is to place an additional photo-detection device having a light source of >600 nm in an expiration conduct of the inhalation apparatus or any other conduct that is affected by the fog created during exhalation but where there is no passage of aerosols. According to this embodiment, only the mist is detected there.

The other photo-detection device(s) having a light source of wavelength <600 nm is/are placed after the valve. The sensor then works as follows: During the passage of aerosols, the one or more from the photo-detection device(s) having a light source of wavelength <600 nm signals reach their limit of detection. When the fog is produced into the mouthpiece, the single photo-detection device having light source of wavelength >600 nm in the exhalation pipe reaches the detection threshold

**c) photo-detection device having a light source of wavelength > 600 nm version 1**

According to a third embodiment, a first sensor with photo-detection device having light source of wavelength > 600 nm is placed in a space which is not reached by the fog but where aerosols flows, for example upstream of the valve 4 of Fig. 1. The signal of this photo-detection device only detects the presence of aerosol. The other second sensor(s) may be placed at the valve outlet to perform the functions for measuring the flow of aerosols and mist detection. The sensors then work as follows: when the fog is produced into the mouthpiece, the signals from the first and second sensors reach the detection threshold, and when the fog is produced into the mouthpiece only the photo-detection device at the valve outlet will reach the threshold of detection.

**d) photo-detection device having a light source of wavelength > 600 nm Version 2**

According to another embodiment, when only one photo-detection device having a light source of wavelength > 600 nm is used, it is placed in an expiration conduit of the inhalation apparatus or any other conduct that is affected by the fog created during exhalation but where there is no passage of aerosols. According to this embodiment, only the mist is detected there. During the passage of aerosols, the one or more from the photo-detection device(s) having a light source of wavelength <600 nm near the output valve reach their limit of detection threshold. When the fog is produced into the mouthpiece, the two photo-detection device reach their detection threshold

According to the present invention, the apparatus is configured to perform a step where the administration of the drug is synchronized (or coordinated) based on the detection of this exhaled mist and/or by measurement of a decrease in the disturbance of the electromagnetic radiation emitted from the sensor caused by inspiration of the subject in the inhalation apparatus, and the corresponding disappearance of this mist formed during exhalation. The display means or audible means of the flow measuring apparatus may also be used to provide instructions to the user, for example, to instruct the use to start inhaling or exhaling, to initiate a coordinated and adequate administration of the drug.

In another embodiment, the method may further comprise the step of measuring a flow of particles. A characteristic signal measured from the flow of particle at the exit of an inhalation chamber (after filtering and amplification of the signal), is presented in Fig. 9A. The detection of the flow of aerosol is measured when the signal value exceeds a threshold rate that depends on the medication administered. As soon as the signal exceeds the threshold rate, an audible or visual notification (or both) informs the patient that he has inhaled the aerosol drug. That is to say, when a velocity of the flow of particles is at or above a threshold rate, the notification is representative of a successful administration; and when a velocity of the flow of particles is below a threshold rate, the notification is representative of an unsuccessful administration. According to the present invention, the respiratory cycle of inspiration/expiration (inhalation and exhalation) is detected with the sensor of the present invention, and is used to time (coordinate, synchronize) the administration of the drug substance of interest. This coordination may be combined with an audible and/or visual signal providing instructions to the user regarding the appropriate action to take to perform the administration.

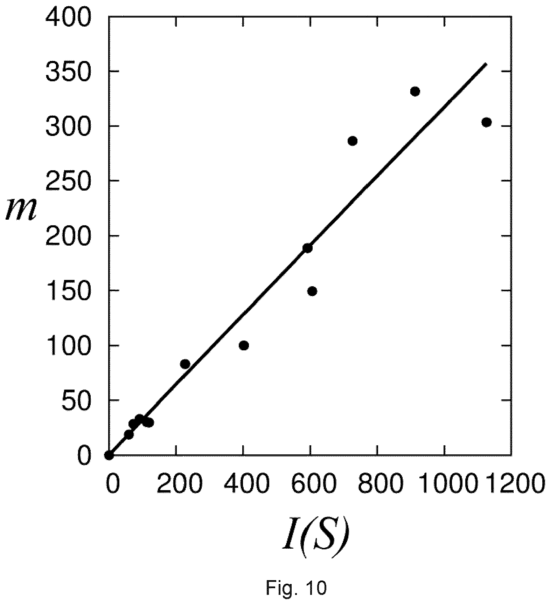

The aerosol mass is correlated to the time integral of the signal. The empirical relationship between mass and the time integral of the signal is obtained from the mass measurement (e.g. measurement on filters or with a cascade impactor) in the laboratory. An example of a calibration curve of the mass as a function of the integral of S (t) signal for a corticosteroid (Fluticasone 50 mcg per dose) is shown in Fig. 10.

**EXAMPLE 1**

**EXAMPLARY FLOW MEASURING APPARATUS**

Photo-detection devices according to the present invention are assembled from infrared emitters (light sources 1 and 2), and photodiodes (detectors 1 and 2). The detectors are operatively coupled to signal filters to eliminate undesired signal frequencies (e.g. a RC high-pass filter at about 3 Hz to eliminate the DC component, and a Butterworth 3 to 10 Hz low-pass filter), and also operatively coupled to an operational amplifier (e.g. MCP602, from Microchip™), mounted to provide a non-inverted gain of 210. Operatively coupled to these component is a controller, for example a micro-controller PIC10LF320 from Microchip™.

## EXAMPLE 2

## MEASURING THE AVERAGE VELOCITY AND MASS OF AEROSOLS

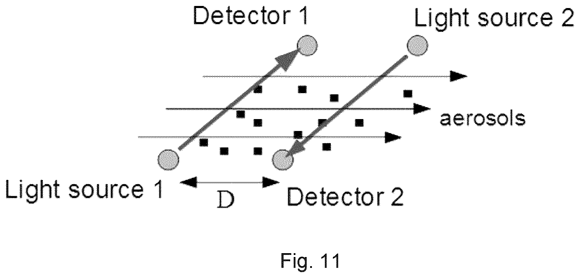

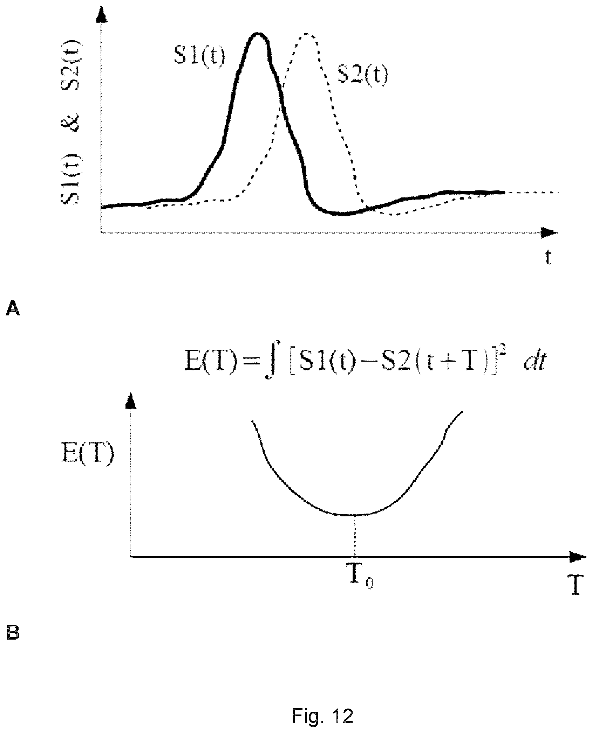

From two photo-detection devices (or more) according to the present invention, described above, measurement of the speed of aerosols can be performed. The velocity calculated is of course an average speed since the speed can vary from one particle to another, depending on the size of the particle and on the detailed local structure of the flow, as explained above. The principle is detailed in the Figs. 11 and 12.

According to this example, the two photo-detection devices, denoted 1 and 2, are arranged in the plane formed by the aerosol jet, perpendicularly to the direction of flow. The light source 1 is arranged on the same side as the detector 2, and the light source 1 is arranged on the same side as detector 1, so that the detectors only receive radiation from their matched light source (Fig. 11). The axes of the light beams (photons) are parallel and separated by a distance D determined with high accuracy. The temporal signals measured by each detector are recorded as S1(t) and S2(t), and have intensity as shown in Fig. 12A. Each signal displays a peak due to the passage of aerosols, each peak being time-shifted relative to the other.

The determination of the time difference between the two signals S1(t) and S2(t) can be traced back to the speed of the aerosols. Shifting the signal S2(t) of a certain duration T brings the two signals in sync, and superimpose them, at least partially. The "error" E(T) is defined as the integral of $(S1(t) - S2(t + T))^2$ (Fig. 12B). The signals may have to be centered or re-scaled to optimize the computation of the error. This integral is approximated by a simple discrete sum. The duration T which minimizes error E(T) is the typical correlation duration, denoted by $T_0$. It allows to estimate the average speed of aerosols by the relationship:

$$average\ speed = \frac{D}{T_0}$$

For the measurement of the mass of particles, calibration is achieved by connecting the apparatus and the sensor to a filter and a respiratory pump. Several streams of drug particles are injected in the inhalation chamber, which comes into contact with the filter, which allows to measure the total mass of aerosol particles that came out of the inhalation chamber. This is carried out as many times as required (e.g. 1 injection, 2 injection, n injection). The total mass measured on the filter is plotted versus the time integral of the signals obtained for each calibration curve, and fitted as a function $m(I) = k\ I^n$. There are as many calibration curves of drugs. Fig. 10 illustrates such a curve for the corticosteroid fluticasone (50 μg per dose)

## Claims

1. An inhalation apparatus (10) comprising an inhalation chamber (2) and a flow measuring apparatus (5) for measuring a flow of particles from an inhalation chamber the inhalation chamber having a first end (20) to be connected to the source (1) of drug, such as a metered drug inhaler, to be administered by inhalation to a user, and a second end (3) to be connected to the user, through a section of the inhalation apparatus, said section being located between the second end of the inhalation chamber and the user, the flow measuring apparatus being located in the section of the inhalation apparatus and comprising:

   • a sensor comprising at least one photo-detection device, each one of the at least one photo-detection device comprising a light source (12) and a detector (14),

   wherein said light source is configured to emit electromagnetic radiation onto said detector along a light path (16), wherein said detector is configured to measure disturbance of said electromagnetic radiation by said flow of particles from the inhalation chamber to provide measurements of the presence of particles within said section of the inhalation apparatus and measurements of a mass of the particles flowing through the inhalation apparatus.

2. The inhalation apparatus of claim 1, wherein the at least one photo-detection device comprises at least two photo-detection devices, wherein the light paths of the at least two photo-detection devices are parallel, and at least one of said light paths is inverted with respect to another one of the light paths.

3. The inhalation apparatus of any one of claims 1 - 2, wherein said sensor is configured to measure said flow of particles along a first axis, further comprising a second sensor configured to measure said flow of particles along a second axis perpendicular to said first axis.

4. The inhalation apparatus of any one of claims 2 - 3, further comprising a processor (30) operatively connected to said sensor for processing a measured disturbance of said electromagnetic radiation; wherein optionally, said processor is operative to provide a rate of drug delivery.

5. The inhalation apparatus of claim 4, wherein said processor is operatively connected to said sensor for controlling one or more of a wavelength, a frequency, or an intensity of the electromagnetic radiation emitted by said light source.

6. The inhalation apparatus of any one of claims 1 - 5, wherein said light source emits at wavelength from 400 nm to 1 mm, and preferably from 450 nm to 600 nm.

7. The inhalation apparatus of any one of claims 2 - 5, wherein one of said photo-detection device has a light source emitting at wavelength from <600 nm and another photo-detection device has a light source emitting at wavelength >600 nm.

8. The inhalation apparatus of any one of claims 2 - 5, further comprising a visual means, operatively connected to said processor, for providing information about said flow of particles;
wherein optionally, said visual means is mounted on said flow measuring apparatus,
wherein optionally said visual means and said processor are configured to provide additional information regarding use of said flow measuring apparatus.

9. The inhalation apparatus of claim 8, wherein said visual means is an indicator light (40), a display means, a detachable display (42), or combinations thereof.

10. The inhalation apparatus of any one of claims 4 - 9, further comprising a transmission means (32), operatively connected to said processor, for transmitting information about said flow of particles to an external device (400).

11. The inhalation apparatus of any one of claims 9 - 10, wherein said display means is a plate comprising a visual information, an illuminated plate comprising a visual information, an LCD display, or a combination thereof.

12. The inhalation apparatus of claim 11, wherein said detachable display is configured to be attached to a separate base (50), wherein optionally said separate base is a powered base, to power said detachable display,
wherein optionally said base is integral to said detachable display.

13. The inhalation apparatus of claim 12, wherein said detachable base further comprise any one of a processor, memory, storage, input interface, and output interface.

14. The inhalation apparatus of claim 4, wherein said processor is configured to detect changes in the measured disturbance of said electromagnetic radiation to determine an occurrence of inhalation or exhalation.

15. The inhalation apparatus of claim 14, wherein said processor is further configured to time an administration of a drug based on the detected changes.

**Patentansprüche**

1. Inhalationsvorrichtung (10), umfassend eine Inhalationskammer (2) und eine Durchflussmessvorrichtung (5) zum Messen eines Stroms von Teilchen aus einer Inhalationskammer, wobei die Inhalationskammer ein erstes Ende (20), das mit der Quelle (1) eines einem Benutzer durch Inhalation zu verabreichenden Medikaments, wie etwa einem Dosier-Medikamenteninhalator, zu verbinden ist, und ein zweites Ende (3) aufweist, das mit dem Benutzer durch einen Abschnitt der Inhalationsvorrichtung zu verbinden ist, wobei der Abschnitt zwischen dem zweiten Ende der Inhalationskammer und dem Benutzer angeordnet ist, wobei die Durchflussmessvorrichtung im Abschnitt der Inhalationsvorrichtung angeordnet ist und umfasst:

   • einen Sensor, der mindestens eine Fotodetektionsvorrichtung umfasst, wobei jede der mindestens einen Fotodetektionsvorrichtung eine Lichtquelle (12) und einen Detektor (14) umfasst,

   wobei die Lichtquelle dafür ausgelegt ist, elektromagnetische Strahlung entlang eines Lichtweges (16) auf den Detektor zu emittieren,
   wobei der Detektor dafür ausgelegt ist, eine Störung der elektromagnetischen Strahlung aufgrund des Stroms von Teilchen aus der Inhalationskammer zu messen, zu dem Zweck, Messungen des Vorhandenseins von Teilchen innerhalb des Abschnitts der Inhalationsvorrichtung und Messungen einer Masse der durch die Inhalationsvorrichtung strömenden Teilchen bereitzustellen.

2. Inhalationsvorrichtung nach Anspruch 1, wobei die mindestens eine Fotodetektionsvorrichtung mindestens zwei Fotodetektionsvorrichtungen umfasst, wobei die Lichtwege der mindestens zwei Fotodetektionsvorrichtungen parallel sind und mindestens einer der Lichtwege in Bezug auf einen anderen der Lichtwege invertiert ist.

**3.** Inhalationsvorrichtung nach einem der Ansprüche 1 bis 2, wobei der Sensor dafür ausgelegt ist, den Strom von Teilchen entlang einer ersten Achse zu messen, und ferner einen zweiten Sensor umfasst, der dafür ausgelegt ist, den Strom von Teilchen entlang einer zweiten, zur ersten Achse senkrechten Achse zu messen.

**4.** Inhalationsvorrichtung nach einem der Ansprüche 2 bis 3, ferner umfassend einen Prozessor (30), der betriebsfähig mit dem Sensor verbunden ist, zu dem Zweck, eine gemessene Störung der elektromagnetischen Strahlung zu verarbeiten;
wobei optional der Prozessor in der Lage ist, eine Medikamentenabgaberate bereitzustellen.

**5.** Inhalationsvorrichtung nach Anspruch 4, wobei der Prozessor betriebsfähig mit dem Sensor verbunden ist, zu dem Zweck, eine oder mehrere Wellenlängen, eine Frequenz oder eine Intensität der von der Lichtquelle emittierten elektromagnetischen Strahlung zu steuern.

**6.** Inhalationsvorrichtung nach einem der Ansprüche 1 bis 5, wobei die Lichtquelle bei einer Wellenlänge von 400 nm bis 1 mm und vorzugsweise von 450 nm bis 600 nm emittiert.

**7.** Inhalationsvorrichtung nach einem der Ansprüche 2 bis 5, wobei eine der Fotodetektionsvorrichtungen eine Lichtquelle aufweist, die bei einer Wellenlänge von <600 nm emittiert, und eine andere Fotodetektionsvorrichtung eine Lichtquelle aufweist, die bei einer Wellenlänge >600 nm emittiert.

**8.** Inhalationsvorrichtung nach einem der Ansprüche 2 bis 5, ferner umfassend ein visuelles Mittel, das betriebsfähig mit dem Prozessor zu dem Zweck verbunden ist, Informationen über den Strom von Teilchen bereitzustellen;
wobei optional das visuelle Mittel an der Durchflussmessvorrichtung montiert ist,
wobei optional das visuelle Mittel und der Prozessor dafür ausgelegt sind, zusätzliche Informationen bezüglich der Verwendung der Durchflussmessvorrichtung bereitzustellen.

**9.** Inhalationsvorrichtung nach Anspruch 8, wobei das visuelle Mittel eine Anzeigeleuchte (40), ein Anzeigemittel, eine abnehmbare Anzeige (42) oder eine Kombination davon ist.

**10.** Inhalationsvorrichtung nach einem der Ansprüche 4 bis 9, ferner umfassend ein Übertragungsmittel (32), das betriebsfähig mit dem Prozessor verbunden ist, zu dem Zweck, Informationen über den Strom von Teilchen an eine externe Vorrichtung (400) zu übertragen.

**11.** Inhalationsvorrichtung nach einem der Ansprüche 9 bis 10, wobei das Anzeigemittel eine Platte ist, die eine visuelle Information umfasst, eine beleuchtete Platte ist, die eine visuelle Information umfasst, eine LCD-Anzeige oder eine Kombination davon ist.

**12.** Inhalationsvorrichtung nach Anspruch 11, wobei die abnehmbare Anzeige dafür ausgelegt ist, an einer separaten Basis (50) befestigt zu werden,
wobei optional die separate Basis eine mit Strom versorgte Basis zum Versorgen der abnehmbaren Anzeige mit Strom ist,
wobei optional die Basis mit der abnehmbaren Anzeige integriert ist.

**13.** Inhalationsvorrichtung nach Anspruch 12, wobei die abnehmbare Basis ferner ein beliebiges Element von einem Prozessor, einem Arbeitsspeicher, einem Massenspeicher, einer Eingabeschnittstelle und einer Ausgabeschnittstelle umfasst.

**14.** Inhalationsvorrichtung nach Anspruch 4, wobei der Prozessor dafür ausgelegt ist, Änderungen der gemessenen Störung der elektromagnetischen Strahlung zu dem Zweck zu erfassen, ein Auftreten von Inhalation oder Exhalation zu bestimmen.

**15.** Inhalationsvorrichtung nach Anspruch 14, wobei der Prozessor ferner dafür ausgelegt ist, die Verabreichung eines Medikaments auf der Grundlage der erfassten Änderungen zeitlich festzulegen.

**Revendications**

**1.** Appareil d'inhalation (10) comprenant une chambre d'inhalation (2) et un appareil de mesure de débit (5) pour mesurer un flux de particules provenant d'une chambre d'inhalation, la chambre d'inhalation ayant une première extrémité (20) à relier à la source (1) de médicament, telle qu'un inhalateur de médicament dosé, à administrer par inhalation à un utilisateur, et une seconde extrémité (3) à relier à l'utilisateur, par l'intermédiaire d'une section de l'appareil d'inhalation, ladite section étant située entre la seconde extrémité de la chambre d'inhalation et l'utilisateur, l'appareil de mesure de débit étant situé dans la section de l'appareil d'inhalation et comprenant :

• un capteur comprenant au moins un dispositif de photodétection, chacun des au moins un dispositif de photodétection comprenant une source de lumière (12) et un détecteur (14),

ladite source de lumière étant configurée pour émettre un rayonnement électromagnétique sur ledit détecteur le long d'un trajet lumineux (16),
ledit détecteur étant configuré pour mesurer la perturbation dudit rayonnement électromagnétique par ledit flux de particules provenant de la chambre d'inhalation pour fournir des mesures de la présence de particules à l'intérieur de ladite section de l'appareil d'inhalation et des mesures d'une masse des particules s'écoulant à travers l'appareil d'inhalation.

2. Appareil d'inhalation selon la revendication 1, l'au moins un dispositif de photodétection comprenant au moins deux dispositifs de photodétection, les trajets lumineux des au moins deux dispositifs de photodétection étant parallèles, et au moins un desdits trajets lumineux étant inversé par rapport à un autre des trajets lumineux.

3. Appareil d'inhalation selon l'une quelconque des revendications 1 et 2, ledit capteur étant configuré pour mesurer ledit flux de particules le long d'un premier axe, comprenant en outre un second capteur configuré pour mesurer ledit flux de particules le long d'un second axe perpendiculaire audit premier axe.

4. Appareil d'inhalation selon l'une quelconque des revendications 2 et 3, comprenant en outre un processeur (30) relié de manière opérationnelle audit capteur pour traiter une perturbation mesurée dudit rayonnement électromagnétique ; en option, ledit processeur étant opérationnel pour fournir un taux d'administration de médicament.

5. Appareil d'inhalation selon la revendication 4, ledit processeur étant relié de manière opérationnelle audit capteur pour commander une ou plusieurs d'une longueur d'onde, d'une fréquence ou d'une intensité du rayonnement électromagnétique émis par ladite source de lumière.

6. Appareil d'inhalation selon l'une quelconque des revendications 1 à 5, ladite source de lumière émettant à une longueur d'onde de 400 nm à 1 mm, et de préférence de 450 nm à 600 nm.

7. Appareil d'inhalation selon l'une quelconque des revendications 2 à 5, l'un desdits dispositifs de photodétection ayant une source de lumière émettant à une longueur d'onde < 600 nm et un autre dispositif de photodétection ayant une source de lumière émettant à une longueur d'onde > 600 nm.

8. Appareil d'inhalation selon l'une quelconque des revendications 2 à 5, comprenant en outre un moyen visuel, relié de manière opérationnelle audit processeur, pour fournir des informations sur ledit flux de particules ; en option, ledit moyen visuel étant monté sur ledit appareil de mesure de débit, en option, ledit moyen visuel et ledit processeur étant configurés pour fournir des informations supplémentaires concernant l'utilisation dudit appareil de mesure de débit.

9. Appareil d'inhalation selon la revendication 8, ledit moyen visuel étant un témoin lumineux (40), un moyen d'affichage, un affichage détachable (42), ou des combinaisons de ceux-ci.

10. Appareil d'inhalation selon l'une quelconque des revendications 4 à 9, comprenant en outre un moyen de transmission (32), relié de manière opérationnelle audit processeur, pour transmettre des informations concernant ledit flux de particules à un dispositif externe (400).

11. Appareil d'inhalation selon l'une quelconque des revendications 9 et 10, ledit moyen d'affichage étant une plaque comprenant des informations visuelles, une plaque éclairée comprenant des informations visuelles, un écran LCD, ou une combinaison de ceux-ci.

12. Appareil d'inhalation selon la revendication 11, ledit affichage détachable étant configuré pour être fixé à une base séparée (50), en option, ladite base séparée étant une base alimentée, pour alimenter ledit affichage détachable, en option, ladite base faisant partie intégrante dudit affichage détachable.

13. Appareil d'inhalation selon la revendication 12, ladite base détachable comprenant en outre l'un quelconque d'un processeur, d'une mémoire, d'un stockage, d'une interface d'entrée et d'une interface de sortie.

14. Appareil d'inhalation selon la revendication 4, ledit processeur étant configuré pour détecter des changements dans la perturbation mesurée dudit rayonnement électromagnétique pour déterminer une occurrence d'inhalation ou d'expiration.

15. Appareil d'inhalation selon la revendication 14, ledit processeur étant en outre configuré pour synchroniser une administration d'un médicament sur la base des changements détectés.

Fig. 1

Fig. 2

17

Fig. 3

Fig. 4

Fig. 5

Fig. 6

42

40
5

A

B

42

5

C

D

Fig. 7

Fig. 8

A

B

Fig. 9

Fig. 10

Fig. 11

A

$$E(T) = \int \left[ S1(t) - S2(t+T) \right]^2 \, dt$$

B

Fig. 12

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- US 62149019
- US 62166225
- US 62233524
- US 20130008436 A